# EUROPEAN PATENT SPECIFICATION

(11) **EP 1 246 930 B1**
(45) Date of publication and mention of the grant of the patent: **09.11.2005**
(21) Application number: 00985689.9
(22) Date of filing: 22.12.2000
(51) Int. Cl.: C12N 15/869, C12N 7/04, A61K 48/00

(54) **REPLICATION INCOMPETENT HERPES VIRUS VECTORS**
REPLIKATIONSUNFÄHIGE HERPESVIRUS-VEKTOREN
REPLICATION DE VECTEURS HERPESVIRUS ATTENUES

(30) Priority: 22.12.1999 GB 9930419
(43) Date of publication of application: 09.10.2002
(73) Proprietor: BioVex Limited, Oxford OX14 4RX (GB)
(72) Inventor: COFFIN, Robert, Stuart, London W1P 6DB (GB)
(74) Representative: Woods, Geoffrey Corlett
(86) International application number: PCT/GB2000/004983
(87) International publication number: WO 2001/046450

(56) References cited:
- WO-A-96/27672
- WO-A-97/13866
- WO-A-97/20935
- WO-A-98/04726
- WF GOINS ET AL.,: "Herpes simplex virus type 1 vector-mediated expression of nerve growth factor protects dorsal root ganglion neurons from peroxide toxicity" JOURNAL OF VIROLOGY, vol. 73, no. 1, January 1999 (1999-01), pages 519-532, XP002164865
- DATABASE BIOSIS [Online] BIOSCIENCES INFORMATION SERVICE, PHILADELPHIA, PA, US; DP WOLFE ET AT.,: "Long term systemic delivery of nerve growth factor for treatment of peripheral neuropathies using replication defective genomic HSV vectors" XP002165708
- DATABASE BIOSIS [Online] BIOSCIENCES INFORMATION SERVICE, PHILADELPHIA, PA, US; MA LEVATTE ET AL.,: "Analysis of a multi-mutant herpes simplex virus type 1 for gene transfer into sympathetic preganglionic neurons and a comparison to adenovirus vectors" XP002164867
- J.A. PALMER ET AL.,: "Development and optimization of herpes simplex virus vectors for multiple long-term delivery to the peripheral nervous system" JOURNAL OF VIROLOGY, vol. 74, no. 12, June 2000 (2000-06), pages 5604-5618, XP002164866
- DATABASE BIOSIS [Online] BIOSCIENCES INFORMATION SERVICE, PHILADELPHIA, PA, US; J. GOSS ET AL.,: "Protection of spinal motor neurons from degeneration following root avulsion in rats by a HSV vector coding human Bcl-2" XP002165709

## Description

### Field of the Invention

The present invention relates to replication incompetent herpes simplex viruses capable of efficiently transferring genes to cells of the peripheral nervous system. It also relates to the use of such viruses in the study and treatment of diseases and conditions of the nervous system.

### Background of the Invention

Herpes simplex viruses 1 and 2 are large DNA viruses the lifecycles of which are characterised by the ability to enter a lifelong latent state in the sensory ganglia of an infected individual from which reactivation of the virus can occur intermittently. During latency the HSV genome, which remains extra-chromosomal, is largely quiescent although the fact that a small part of the genome encoding the latency associated transcripts (LATs) is transcribed during latency (Stevens, 1987) shows that the development of gene delivery vectors allowing transgene expression during this time should be possible. During latency HSV is not naturally cleared by the immune system and infected cells remain undamaged, further showing the potential for a long-term therapeutic benefit using HSV as a vector (reviewed by Coffin and Latchman 1996, Fink 1996). HSV also has the unique ability among viruses currently under development as vectors to infect axonal nerve terminals followed by efficient retrograde axonal transport of the virus to the cell body in the spinal ganglia. Thus HSV vectors might be developed allowing peripheral vector inoculation to give gene delivery to the spinal ganglia following such retrograde transport. There are a number of potential applications for vectors capable of axonal transport in the peripheral nervous system, including the stimulation of regrowth of damaged nerves, the study/treatment of various pain states, the protection of neurons from further degeneration in e.g. motor neuron disease, the study/treatment of various neuropathies, the study of neuronal development, and the screening of the relevance of genes implicated as being important in any of these processes by a gene delivery approach.

Previous work has shown that HSV vectors can deliver genes to spinal ganglia by such routes, but this in each case has used vectors retaining at least same replication competence as this has been thought necessary for effective gene delivery. Here various mutant viruses have been used variously mutated for the non-essential genes encoding ICP6 (Goldstein and Weller 1988), ICP0 (Leib *et al*. 1989), TK (Palella *et al*. 1988, Ho and Mokarski 1988), gC (Lokensgard *et al*. 1994; Dobson *et al*. 1995; Goins *et al*. 1994), vmw65 (Ecob Prince *et al*. 1995), un-disabled (Margolis *et al*. 1993, Lachman *et al*. 1996, Lachmann and Efstathiou, 1997), or deleted for ICP34.5 or ICP34.5 and vmw65 (Coffin *et al*. 1996). Each of these viruses is attenuated compared to wild type virus even though the mutated genes are non-essential, and each have shown reasonable levels of at least short term gene delivery and/or the ability to enter latency following inoculation of animals by the footpad or ear pinna routes. As discussed however, all retain at least same degree of replication competence in vivo and in culture as no essential genes have been inactivated. Therefore, while replication incompetent HSV vectors have been available for some time, none have been used in the peripheral nervous system as it has so far been assumed that viruses incapable of replication in any cell (i.e. mutated for an essential gene/genes) would not allow gene delivery to spinal ganglia following inoculation by peripheral routes.

### Summary of the Invention

We have previously reported that HSV vectors mutated for ICP34.5 or ICP34.5 and vmw65 can give gene delivery to the peripheral nervous system following footpad inoculation (Coffin *et al*., 1996). However, similar to other work discussed above, such viruses while attenuated still retain some degree of replication competence *in vivo.* Indeed it had been assumed that such replication competence might be necessary for gene delivery by such a route as the virus is required to traffic from the point of inoculation to the cell body in the spine where the delivered gene is to be expressed. Replication might be imagined to be necessary for this to occur efficiently. Therefore while promising results of gene delivery to the peripheral nervous system have been reported, including the alteration of sensitivity to painful stimuli by an HSV-vector mediated approach (Wilson *et al*. 1999), the vectors used retain replication competence raising issues of safety and acceptability if they are to be used for human treatment. When such viruses carry an exogenous gene or genes, issues of safety and acceptability may also arise when they are used in laboratory based pre-clinical and basic research. The present invention describes vectors which are highly efficient at gene delivery to the peripheral nervous system following peripheral inoculation and which have a considerably improved safety/acceptability profile over vectors which have previously been available for such a purpose.

In summary, we have made the surprising finding that highly disabled HSV vectors which cannot replicate in any cell other then those used to prepare vector stocks (i.e. essential gene/genes have been inactivated in the vector which are complemented in the producer cell line) can give highly efficient gene delivery to the peripheral nervous system following direct injection into a peripheral nerve. Such a result, giving very high efficiency gene delivery, has not previously been reported and thus HSV vectors which show an improved safety/acceptability profile over those which have previously been used in the peripheral nervous system are provided by the current invention. Such replication incompetent herpes virus vectors may be used in methods of studying or treating disorders of the peripheral nervous system. In particular, such vectors may be used in methods of target validation, for example by screening genes to identify what, if any, role they play in a peripheral nervous system disorder.

Accordingly, the present invention provides:
- use of a replication incompetent herpes virus which:
   (i) lacks at least one functional immediate early gene selected from genes encoding ICP4 and ICP27;
   (ii) lacks a functional VMW65 gene due to a mutation in said gene which abolishes its transcriptional-activation activity; and
   (iii) comprises a heterologous gene operably linked to the LAT P2 region and a non-LAT promoter;
   in the manufacture of a medicament for use in treating or preventing a peripheral nervous system disorder by administering said medicament to a peripheral nerve by direct injection into said nerve; and
- a method of studying a peripheral nervous system disorder in a non-human animal which method comprises monitoring an effect of expression of a heterologous gene in a peripheral nerve, wherein said heterologous gene has been delivered to said nerve by direction injection of a replication incompetent herpes virus as defined above into said nerve, wherein said gene is implicated in a peripheral nervous system disorder and wherein said disorder is selected from motor neuron disease, pain, peripheral nerve damage and a neuropathy.

### Detailed Description of the Invention

### Viruses

The present invention provides a method for identifying a gene that may be used as a target for therapeutic intervention in the treatment or prevention of a peripheral nervous system disorder. Such genes are identified by expressing a test gene in a cell of the peripheral nervous system of a non-human animal using a replication incompetent herpes virus vector to deliver the gene to the cell and by determining whether expression of the gene affects a phenotype associated with a peripheral nervous system disorder. A method of determining whether a gene influences a phenotype associated with a peripheral nervous system disorder consists essentially of the following steps:
(i) inoculating a peripheral nerve of a non-human animal *in vivo* or *in vitro* with a replication incompetent herpes virus capable of efficiently infecting a cell of the peripheral nervous system, which virus comprises a test gene;
(ii) monitoring a phenotype of said disorder to determine thereby whether expression of said gene has an effect on said phenotype.

The present invention provides a method for screening genes to identify a gene which is important in a peripheral nervous system disorder and which may be used as a target for identifying agents for treating the disorder.

The present invention also provides the use of a replication incompetent herpes virus vector in the manufacture of a medicament for the treatment of a peripheral nervous system disorder. The herpes virus vector is typically replication incompetent and is capable of efficiently infecting a cell of the peripheral nervous system. Preferably the herpes virus vector comprises a heterologous gene. The heterologous gene typically comprises a polypeptide of therapeutic use.

Administration of the herpes virus vector to a subject suffering from disorder of the peripheral nervous system typically alleviates the symptoms of the disorder an/or prevents the progression of the disorder.

A virus for use in the invention is capable of efficiently infecting a cell of the peripheral nervous system following peripheral inoculation, but is incapable of replicating in any cell unless at least one gene which has been rendered non-functional in the virus is also expressed in the cell, i.e. the virus is replication incompetent other than in the cells in which virus stocks are prepared. A virus is replication incompetent if it is incapable of replicating in a cell which does not express at least one functional essential viral gene.

A virus capable of efficiently infecting a cell of the peripheral nervous system following peripheral inoculation typically infects at least 50 cells for example, at least 60, at least 70, at least 80, at least 90 or at least 100 cells, more preferably at least 200, for example at least 500 or at least 1000 cells following direct injection of virus at a titre of from 1 x 1 x 10⁸ pfu/ml to 1 x 10⁹ pfu/ml, preferably from 3 x 10⁸ pfu/ml to 7 x 10⁸ pfu/ml, more preferably from 4 x 10⁸ pfu/ml to 6 x 10⁸ pfu/ml, more preferably 5 x 10⁸ pfu/ml into a peripheral nerve, for example the sciatic nerve, of a laboratory animal. This can be determined by monitoring the expression heterologous marker gene such as LacZ or GFP operably linked to a control sequence, preferably a control sequence which is active during herpes virus latency. Preferably the peripheral nervous system cells are dorsal root ganglion (DRG) cells. Preferably the laboratory animal is a primate or a rodent such as a rat or a mouse.

By definition such viruses are attenuated such that they are replication incompetent. A virus may be rendered replication incompetent by the mutation of at least one essential gene such that the essential gene is non-functional. Viral regions altered for the purposes of such attenuation may be eliminated (completely or partly), made non-functional, or substituted by other sequences, in particular by a heterologous gene sequence. Attenuating mutations resulting in a replication incompetent virus have been described for all neurotrophic herpes viruses so far described. Replication incompetent viruses are generally capable of replicating in cell lines which express a functional essential viral gene in which compensates for the absence of a functional essential viral gene in the replication incompetent virus. Such cell lines may be used to prepare viral stocks.

Although the present invention has been exemplified using herpes simplex viruses, it will be understood that other neurotrophic viruses of the herpesviridae family may be modified such that they are replication incompetent and can achieve efficient gene delivery to cells of the peripheral nervous system following peripheral inoculation. In particular, such viruses may include varicella zoster virus, pseudo-rabies virus or bovine herpes viruses. In addition, herpes amplicon vectors are replication incompetent herpesviruses. Thus, herpes amplicon vector stocks which are capable of infecting cells of the peripheral nervous system following peripheral nerve inoculation may also be used in accordance with the present invention.

When the virus of the invention is a herpes simplex virus, the virus may be derived from, for example, HSV1 or HSV2 strains, or derivatives thereof, preferably HSV1. Derivatives include inter-type recombinants containing DNA from HSV1 and HSV2 strains. Such inter-type recombinants are described in the art, for example in Thompson *et al*. 1998 and Meignier *et al*. 1988. Derivatives preferably have at least 70% sequence homology to either the HSV1 or HSV2 genomes, more preferably at least 80%, even more preferably at least 90 or 95% and most preferably at least 96, 97, 98 or 99%. More preferably derivatives have at least 70% sequence identity to either a HSV1 or HSV2 genome, more preferably at least 80%, even more preferably at least 90 or 95% and most preferably at least 96, 97, 98 or 99%.

A derivative may have the sequence of a HSV1 or HSV2 genome modified by nucleotide substitutions, for example from 1,2 or 3 to 10, 25, 50 or 100 substitutions. The HSV1 or HSV2 genome may alternatively or additionally be modified by one or more insertions and/or deletions and/or by an extension at either or both ends.

For example the UWGCG Package provides the BESTFIT program which can be used to calculate homology (for example used on its default settings) (Devereux *et al*. (1984) *Nucleic Acids Research* **12**, p387-395). The PILEUP and BLAST algorithms can be used to calculate homology or line up sequences (typically on their default settings), for example as described in Altschul (1993) J. Mol. Evol. 36:290-300; Altschul *et al*. (1990) J. Mol. Biol. 215:403-10.

Software for performing BLAST analyses is publicly available through the National Centre for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al*., 1990). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) *Proc. Natl. Acad. Sci.* USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) *Proc. Natl. Acad Sci.* USA 90: 5873-5787. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

Viruses of the invention may be functionally inactive for individual (e.g. ICP27 or ICP4) or multiple (ICP27 and/or ICP4 and/or ICP22 and/or ICP0 and/or ICP47) immediate early genes. They may additionally contain a mutation in the gene encoding vmw65 reducing/preventing its transcriptional activation capability. Alternatively essential genes which are not immediate early genes, such as essential genes involved in virus replication, for example the products of UL29 and UL30 genes, single stranded DNA binding protein and DNA polymerase respectively, may be deleted either alone, in any combination, or in combination also with the inactivation of one or more immediate early genes and/or vmw65. Examples of such viruses include viruses reported by Samaniego *et al*. 1998, Krisky *et al*. 1998, or Thomas *et al*. 1999. Particularly preferred viruses are those in which immediate early gene expression has been prevented or minimised for example, a virus with inactivating mutations in or deletions of deletions of the genes encoding ICP4. ICP27, ICP0 and ICP22 is preferred. Viruses with inactivating mutations in or deletions of ICP27 and/or ICP4 and also such a virus with a mutation in vmw65 reducing/preventing its trans-activating activity are also preferred.

Preferably viruses of the invention do not express, or express only very low levels of at least two immediate early genes. Very low levels are levels below the detection limit of a western blot using an antibody to a protein product of an immediate early gene. A virus may comprise any suitable mutation which minimises immediate early gene expression. Preferably a virus does not express, or expresses only very low levels of at least three immediate early genes. More preferably a virus does not expresses, or expresses only very low levels of at least four immediate early genes. The immediate early genes which must not be expressed or be expressed at only minimal levels are ICP0, ICP4, ICP22 and ICP27. The two immediate early genes may be ICP27 and ICP4, ICP27 and ICP0, ICP27 and ICP22, ICP4 and ICP0, ICP4 and ICP22 or ICP0 and ICP22.

Combinations of gene deletions useful in accordance with the present invention are those in which mutations not only prevent replication but which also minimise or reduce toxicity of the virus. Thus a virus capable of infecting a cell of the peripheral nervous system following peripheral inoculation and with mutations in ICP4 and /or ICP27 is particularly preferred. These mutations prevent expression of not only a functional form of the mutated gene itself but also other viral genes that may be cytotoxic. In addition, viruses with mutations in combinations of or in individual essential genes which can be efficiently complemented by a cell line prepared for their growth are preferred. In particular high titres of the virus can readily be prepared. Such virus cells lines are described in the art, e.g. Samaniego et al 1998 and Krisky et al 1998. The terminology used in describing the various HSV genes is as found in Coffin and Latchman, 1996.

### Production of Replication Incompetent Viruses

When the herpes simplex viruses of the invention lack a particular functional essential gene, for example a gene encoding ICP4 or ICP27, the virus of the invention is propagated on a cell line expressing that essential gene. For example, when the virus lacks a functional ICP27 gene, the virus may be propagated on V27 cells (Rice and Knipe, 1990), 2-2 cells (Smith *et al*., 1992) or B 130/2 cells (Howard *et al*. 1998). When the virus lacks a functional ICP4 gene the virus may be propagated on a cell line expressing ICP4, for example E5 cells (DeLuca *et al*., 1985). When the virus lacks a functional ICP4 gene and a functional ICP27 gene the virus is propagated on a cell line expressing both ICP4 and ICP27 (such as E26 cells; Samaniego *et al*., 1995), and when the virus additionally lacks a functional vmw65 gene the virus may be propagated on a cell line also containing a non-HSV homologue of vmw65 (e.g. from equine herpes virus as in Thomas *et al*. 1999). Mutations to vmw65 may also be partially compensated for by inclusion of hexamethylene bisacetamide (HMBA) in the media used for virus growth (MacFarlane *el al*. 1992).

ICP27-expressing cell lines can be produced by co-transfecting mammalian cells, for example the Vero or BHK cells, with a vector, preferably a plasmid vector, comprising a functional HSV ICP27 gene capable of being expressed in said cells, and a vector, preferably a plasmid vector, encoding a selectable marker, for example neomycin resistance. Clones possessing the selectable marker are then screened further to determine which clones also express functional ICP27, for example on the basis of their ability to support the growth of ICP27- HSV strains, using methods known to those skilled in the art (for example as described in Rice and Knipe, 1990). Cell lines which do not allow reversion of an ICP27- mutant HSV strain to a strain with functional ICP27 are produced as described above, ensuring that the vector comprising a functional ICP27 gene does not contain sequences that overlap with (i.e. are homologous to) sequences remaining in the ICP27- mutant virus.

Where HSV strains of the invention comprise inactivating modifications in other essential genes, for example ICP4, complementing cell lines will comprise a functional HSV gene which complements the modified essential gene in the same manner as described for ICP27. For example in the case of HSV strains comprising mutations in both ICP27 and ICP4, a cell line expressing both ICP27 and ICP4 is used such as E26 cells (Samaniego *et al*., 1995). HSV strains expressing other essential genes can be constructed in a similar manner to that described for ICP27. Here again, if it is ensured that there is no sequence overlap between the remaining virus DNA and that inserted into the cell line for virus growth, the possibility of reversion of the virus to a less disabled form during growth will be minimised.

### Methods of mutation

The various viral genes referred to may be rendered functionally inactive by any suitable technique. For example, they may be rendered functionally inactive by deletions, substitutions or insertions, preferably by deletion. Deletions may remove portions of the genes or the entire gene. For example, deletion of only one nucleotide may be made, resulting in a frame shift. However, preferably larger deletions are made, for example at least 25%, more preferably at least 50% of the total coding and non-coding sequence (or alternatively, in absolute terms, at least 10 nucleotides, more preferably at least 100 nucleotides, most preferably, at least 1000 nucleotides). It is particularly preferred to remove the entire gene and same of the flanking sequences. Inserted sequences may include the heterologous genes described below. In particular, it is preferred to insert the heterologous gene into ICP27 or ICP4. In the case of the vmw65 gene, the entire gene is not deleted since it encodes an essential structural protein, but a small inactivating mutation is made which abolishes the ability of VMW65 to activate transcriptionally IE genes (e.g. as in Ace *et al*., 1989 or Smiley *et al*. 1997).

Mutations are made in the herpes viruses by homologous recombination methods are well known to those skilled in the art. For example, HSV genomic DNA is transfected together with a vector, preferably a plasmid vector, comprising the mutated sequence flanked by homologous HSV sequences. The mutated sequence may comprise deletions, insertions or substitutions, all of which may be constructed by routine techniques. Insertions may include selectable marker genes, for example lacZ or GFP, for screening recombinant viruses by, for example, β-galactosidase activity or fluorescence.

### Heterologous genes and promoters

The viruses for use in the invention are typically modified to carry a heterologous gene. The term "heterologous gene" encompasses any gene. A heterologous gene may be a test gene for use in a method of target validation or a gene of therapeutic use for use in a method of treatment or in the manufacture of a medicament. Although a heterologous gene is typically a gene not present in the genome of a herpes virus, a herpes gene may be used provided that the coding sequence is not operably linked to the viral control sequences with which it is naturally associated.

The heterologous gene may be any allelic variant of a wild-type gene, or it may be a mutant gene. The term "gene" is intended to cover nucleic acid sequences which are capable of being at least transcribed. Thus, sequences encoding mRNA, tRNA and rRNA are included within this definition. However, the present invention is primarily concerned with the expression of polypeptides and/or mRNA and antisense RNA rather than tRNA and rRNA. The term "gene" covers a polynucleotide comprising the coding sequence of a polypeptide. The polynucleotide may include one or more introns, for example may comprise genomic DNA. Preferably the polynucleotide comprises cDNA. Sequences encoding mRNA will optionally include some or all of 5' and/or 3' transcribed but untranslated flanking sequences naturally, or otherwise, associated with the translated coding sequence. It may optionally further include the associated transcriptional control sequences normally associated with the transcribed sequences, for example transcriptional stop signals, polyadenylation sites and downstream enhancer elements.

A heterologous gene may be inserted into the viral genome by homologous recombination of HSV strains with, for example, plasmid vectors carrying the heterologous gene flanked by HSV sequences. The heterologous gene may be introduced into a suitable plasmid vector comprising herpes viral sequences using cloning techniques well-known in the art. The heterologous gene may be inserted into the viral genome at any location provided that the virus can still be propagated. It is preferred that the heterologous gene is inserted into an essential gene. Heterologous genes may be inserted at multiple sites within the virus genome.

The transcribed sequence of a heterologous gene is preferably operably linked to a control sequence permitting expression of the heterologous gene in a mammalian cell, preferably a cell of the peripheral nervous system. The term "operably linked" refers to a juxtaposition wherein the components described are in a relationship permitting them to function in their intended marmer. A control sequence "operably linked" to a coding sequence is joined in such a way that expression of the coding sequence is achieved under conditions compatible with the control sequence.

Typically the heterologous gene is under the control of a promoter active during herpes virus latency. Such promoters typically comprise fragments of the latency associated transcript (LAT) region of the virus or are chimaeric promoters comprising fragments of the LAT region together with a non-LAT promoter. Such latently active promoters may drive the expression of endogenous gene or genes from within the LAT region of the virus, or when inserted at an ectopic, non-LAT site within the HSV genome.

The control sequence comprises a promoter allowing expression of the heterologous gene and a signal for termination of transcription. The promoter is selected from promoters which are functional in mammalian, preferably rat, mouse, guinea pig, ferret or human, cells of the peripheral nervous system. The promoter/promoters may be derived from promoter sequences of eukaryotic genes. For example, promoters may be derived from the genome of a cell in which expression of the heterologous gene is to occur, preferably a mammalian, preferably human cell of the peripheral nervous system. With respect to eukaryotic promoters, they may be promoters that function in a ubiquitous manner (such as promoters of β-actin, tubulin) or, alternatively, a tissue-specific manner, such as the neuron-specific enolase (NSE) promoter. They may also be promoters that respond to specific stimuli, for example promoters that bind steroid hormone receptors. Viral promoters may also be used, for example the Moloney murine leukemia virus long terminal repeat (MMLV LTR) promoter or other retroviral promoters, the human or mouse cytomegalovirus (CMV) IE promoter, or promoters of herpes virus gene including those driving expression of the latency associated transcripts.

Expression cassettes and other suitable constructs comprising a heterologous gene and control sequences can be made using routine cloning techniques known to persons skilled in the art (sec, for example, Sambrook *et al*., 1989, Molecular Cloning - A Laboratory Manual; Cold Spring Harbor Press).

It may also be advantageous for the promoters to be inducible so that the levels of expression of the heterologous gene can be regulated during the life-time of the cell. Inducible means that the levels of expression obtained using the promoter can be regulated. For example, in a preferred embodiment where more than one heterologous gene is inserted into the HSV genome, one promoter would comprise a promoter responsive to the tet repressor/VP16 transcriptional activator fusion protein previously reported (Gossen and Bujard, 1992, Gossen *et al*., 1995), and driving the heterologous gene the expression of which is to be regulated. The second promoter would comprise a strong promoter (e.g. the CMV IE promoter) driving the expression of the tet repressor/VP16 fusion protein. Thus in this example expression of the first heterologous gene would depend on the presence or absence of tetracycline.

In preferred embodiments of the current invention, a promoter is used which allows gene expression during herpes virus latency. Such promoters may be derived from the LAT region (e.g. LAP2; Goins *et al*. 1994) and may be chimaeric promoters containing sequences from the LAT region fused to a LAT or non-LAT promoter or other elements (e.g. Lachmann and Efstathiou, 1997). These promoters may function from within the endogenous LAT region of the virus or when inserted at a heterologous site in the virus genome. In particularly preferred embodiments chimaeric promoters consisting of a non-LAT promoters such as the CMV, MMLV or NSE promoters, or fragments thereof, linked to LAT-derived sequences, including those present in LAP 1 (Goins *et al*. 1994), LAP2 (Goins *et al*. 1994) and/or LAT P2 are used. The LAT P2 region is here defined as HSV1 nucleotides 118866-120219 of HSV strain 17+ (GenBank HE1CG: from PstI-BstXI sites), and includes fragments or derivatives of this region, including homologous regions of other HSV1 strains and of HSV2 strains, which are capable of providing a long-term expression capability to promoters to which they are linked. Such chimaeric promoters may also allow the expression of multiple heterologous genes by placing pairs of promoters away from a central LAT-derived element such that two genes may be expressed from a single expression cassette (e.g. as in Thomas *et al*. 1999).

Heterologous genes in viruses for therapeutic uses will typically encode polypeptides of therapeutic use. For example, genes which may modulate pain, stimulate nerve re-growth or prevent nerve degeneration. Heterologous genes may be wild-type genes or may be mutated to enhance the function of the gene or to inhibit function of the gene. For example, where an endogenous gene is over expressed in a peripheral nervous system disorder, a non-functional gene which disrupts the function of the endogenous gene may be useful in treating the disorder.

In particular, the heterologous gene/genes may encode a polypeptide capable of modifying responses to painful stimuli or reducing chronic pain, for example a protein capable sequestering, for example nerve growth factor, other pain modulating neurotrophic factor or neurotrophic factor-like molecules, or substance P or other neuropeptides. The heterologous gene/genes may also encode a polypeptide capable of stimulating the regrowth of damaged nerves or preventing the further degeneration of nerves in degenerative conditions.
Suitable heterologous genes include growth factors such as NT-3, NT-4, NT-5, Reg-2, CNTF, GDNF, BDNF, NGF, TrkA and TrkB, neuropeptides such as substance P and galanin, anti-apoptotic genes such as Bcl2 and pain modulators such as enkephalin.

Heterologous genes may also include marker genes (for example encoding p-galactosidase or green fluorescent protein or other fluorescent proteins) or genes whose products regulate the expression of other genes (for example, transcriptional regulatory factors including the tet repressor/vmw65 transcriptional activator fusion protein described above).

Target validation, gene therapy, and other therapeutic applications may well require the administration of multiple genes. The expression of multiple genes may be advantageous for the treatment of a variety of conditions. Herpes viruses are uniquely appropriate as they do not have the limited packaging capabilities of other viral vector systems. Thus multiple heterologous genes can be accommodated within its genome. There are, for example, at least two ways in which this could be achieved. For example, more than one heterologous gene and associated control sequences could be introduced into a particular HSV strain either at a single site or at multiple sites in the virus genome. It would also be possible to use pairs of promoters (the same or different promoters) facing in opposite orientations away from each other, these promoters each driving the expression of a heterologous gene (the same or different heterologous gene) as described above.

A virus may comprise one, two, three, four or more heterologous genes. Where the gene is a test gene for use in a method of target validation, the virus typically comprises one heterologous gene. The virus may comprise one, two or more copies of a heterologous gene. A virus for use in a method of target validation may comprise more than one heterologous test gene. This will enable several genes, for example from 2 to 20 genes, preferably from 2 or 4 to 10 genes, to be screened simultaneously, or interactions between genes under test to be examined. If an effect of a test gene is observed, further viruses carrying fewer test genes, for example one or two test genes, may be used to determine which of the genes is responsible for the observed effect.

Heterologous genes for use in target validation methods will typically be suspected to be of importance in a peripheral nervous system disorder. For example, a gene may be implicated in a disorder due to its presence on region of a chromosome linked to the disorder. Alternatively a gene may be implicated in a peripheral nervous system disorder if it is known to be expressed in the peripheral nervous system. A gene may be implicated in a peripheral nervous system disorder particularly if it is upregulated or down-regulated in a peripheral nervous system cell or in tissue innervated by a peripheral nervous system cell during a natural, artificial or induced disease state or following nerve damage or following administration of a stimuli inducing an acute or chronic pain state or other stimuli which induces a dysfunction in or alteration to the normal functioning of the peripheral nervous system or a peripheral nerve cell.

### Monitoring Phenotype

As used herein the term "phenotype" is used to refer to any physical manifestation of a disorder of the central nervous system. The term "phenotype" is thus intended to include changes at the molecular, cellular, tissue, cognitive and behavioural levels. Damage and trauma to the central nervous system, for example as a result of physical and chemical injury is also included within the term "phenotype".

Any suitable phenotype associated with a peripheral nervous system disorder may be monitored. For example, the sensitivity of an animal to pain may be monitored using any suitable assay for monitoring the behavioral response of an animal to pain stimuli. A test gene which influences pain sensitivity will typically enhance or diminish the response of the animal to a given stimulus. Control responses may be determined by testing an animal prior to inoculation with the virus or by testing one or more control animals. A control animal is an animal which has not been inoculated with a virus or which has been inoculated with a control virus which does not express a heterologous gene or which expresses a heterologous gene that does not affect the phenotype of interest. A phenotype associated with a peripheral nervous system disorder may also be monitored at the cellular or molecular level. This is particularly important when monitoring the effect of a test gene *in vitro,* for example following isolation of peripheral nervous system cells which have been transduced in vivo following peripheral inoculation with a virus of the invention. For example, the cellular response to the application of a chemical stimulus such as capsaicin may be monitored. This may be achieved using electrophysiological techniques. Alternatively any changes in gene expression within a peripheral nervous system cell in response to the test gene may be monitored, as may responses to stimuli which might otherwise induce cell death.

A test gene may be suitable as a target for gene therapy or for small molecule modulators useful in the treatment of a peripheral nervous system disorder if it influences a phenotype associated with the disorder. A gene identified as a target using a method of the invention may be used in a method of screening for agents useful in the treatment of a peripheral nervous system disorder if it influences a phenotype associated with the disorder. Generally a test gene may be considered to influence a phenotype if it inhibits or enhances the phenotype, for example expression of a phenotype may be increased or decreased by at least 5%, for example by at least 10%, at least 15%, at least 20% or at least 25%, preferably by at least 30%, for example at least 40% or at least 50%, more preferably by at least 70%, for example, at least 80% or at least 90% compared to controls.

### Inoculation of a peripheral nerve

A replication incompetent herpes virus is typically inoculated into a peripheral nerve by direct application to the nerve, for example by injection. Generally the virus is applied at titres of from 10⁴ to 10¹⁰ pfu, preferably from 10⁷ to 10¹⁰, more preferably at least 10⁸ pfu. Typically from 1µl to 20µl, preferably from 2µl to 10µl, more preferably from 2µl to 5µl of virus suspension is injected.

Any peripheral nerve, for example the sciatic, femoral, ulnar, median or radial nerves may be injected with the virus. The nerve is preferably a mammalian nerve, for example a rodent such as a mouse or a rat, a primate such as monkey or a weasel or a pig nerve. The nerve may be present in a non-human animal which models a peripheral nervous system disorder. The animal may be a transgenic animal.

### Therapeutic uses

Replication incompetent herpes viruses capable of infecting cells of the peripheral nervous system as described herein may be used in methods for treating or preventing peripheral nervous system disorders. As used herein, the term "disorder" is intended to include any condition of the nervous system which affects the normal functioning of the peripheral nervous system. "Conditions", "diseases" and "injury" to the peripheral nervous system are thus encompassed by the term "disorder". The term "disorder" also includes animal models of peripheral nervous system disorders. In particular, viruses of the invention may be used for the treatment of pain states or for the stimulation of nerve regrowth or to prevent nerve degeneration.

The herpes viruses of the present invention may thus be used to deliver therapeutic genes to a human or animal in need of treatment. Delivery of therapeutic genes using the herpes viruses of the invention may be used to treat peripheral nervous system disorders, for example pain, degenerative conditions or to stimulate nerve regrowth. Generally, the condition of a patient suffering from a peripheral nervous system disorder is improved by administration of the virus.

One method of administering therapy involves inserting a therapeutic gene into the genome of the herpes virus of the invention, as described above, and then combining the resultant recombinant virus, as active ingredient, with a pharmaceutically acceptable carrier or diluent to produce a pharmaceutical composition. Suitable carriers and diluents include isotonic saline solutions, for example phosphate-buffered saline.

Gene delivery to cells of the peripheral nervous system may be carried out by direct injection of the vector composition into a peripheral nerve of a human or animal. The amount of virus administered is in the range of from 10⁴ to 10'° pfu, preferably from 10⁵ to 10⁸ pfu, more preferably about 10⁶ to 10⁸ or 10⁹ pfu, or from 10⁸ to 10¹⁰ pfu. When injected, typically from 1-200 µl preferably from 1 to 10 µl of virus suspension, depending on the species and nerve, in a pharmaceutically acceptable suitable carrier or diluent, is administered.

The routes of administration and dosages described are intended only as a guide since a skilled practitioner will be able to determine readily the optimum route of administration and dosage. The dose of a modulator may be determined according to various parameters, especially according to the substance used; the age, weight and condition of the patient to be treated; the route of administration; and the required regiment. A physician will be able to determine the required dosage for any particular patient.

The invention will be described with reference to the following Examples, which are intended to be illustrative only and not limiting.

### EXAMPLES

### Materials and Methods

### Virus Strains

Replication incompetent virus strains are derived from HSV1 strain 17+, the nucleotide sequence of which is deposited in GenBank (Accession No. HE1CG). These can be propagated on 27/12/M:4 cells which are BHK cells containing the HSV ICP27 and ICP4-encoding genes and equine herpes virus gene 12 (Thomas *et al*., 1999). These cells allow efficient growth of viruses with mutations in ICP4, ICP27 and with mutations in vmw65 reducing its transactivating activity. Viruses not containing mutations in ICP27 but having mutations in ICP4 and vmw65 can be propagated in B4/2 cells which are similar to 27/12/M:4 cells but do not contain the ICP27-encoding gene. Alternatively B 130/2 cells can be used, either with or without HMBA, which complement mutations to only ICP27 (Howard *et al*. 1998) for the growth of viruses not including mutations to ICP4.

The current invention was exemplified using virus strain 1764/27-/4-/pR191acZ and 1764/4-pR19lacZ. The first virus is deleted for ICP34.5, ICP27 and ICP4 with an inactivating mutation in vmw65 as previously described for virus strain 1764/27-/4-/pR20.5 (Thomas *et al*. 1999). The second virus is identical to the first except that it is not deleted for ICP27. These viruses contains a CMV promoter (from pcDNA3; Invitrogen)/lacZ (pCH110; Pharmacia)/poly A (from pcDNA3) cassette inserted into both copies of the LAT region between the BstXI sites at nts 120,219 and 120,413 rather than the insertion into ICP4 described in Thomas *et al*. 1999. Virus strain 1764/27-/4-/pR19GFP is identical to virus strain 1764/27-/4-/pR19lacZ except that the lacZ gene has been directly replaced by the GFP gene (Clontech). Other virus strains 17+/pR191acZ (Wagstaff *et al*. 1998; ICP27 deleted) and 1764/27-/pR20.5/vhs (ICP34.5, ICP27 deleted, inactivating mutation in vmw65) were also used in initial footpad experiments.

### Example 1. Replication incompetent HSV gives only low efficiency gene delivery to cells of the peripheral nervous system following footpad inoculation.

Balb/c mice were inoculated by the footpad route as previously described (Coffin *et al*. 1996) with 20µl of virus strains 1764/27-/4-/pR191acZ, 17+/pR191acZ or 1764/27-/pR20.5/vhs at titres of 1 x 10⁷pfu/ml, 1x10⁸pfu/ml and 5x 10⁸pfu/ml. DRGs were removed 1 week after inoculation and fixed and stained with X-gal as previously described (Coffin *et al*. 1996). In contrast to results previously obtained with replication competent viruses with mutations in ICP34.5 or vmw65 and ICP34.5 (Coffin *et al*. 1996), very little gene expression was seen at any of the viruses at any of the titres tested. However with 1764/27-/4-/pR191acZ a maximum of 30 X-gal staining cells was seen at the highest titre used compared to a maximum 5 cells with the less disabled viruses which were also tested. Therefore replication incompetent HSV cannot be used for efficient gene delivery to dorsal root ganglion cells following footpad inoculation in mice although the most highly disabled replication incompetent vector gave slightly improved results compared to the other replication incompetent vectors tested.

### Example 2. Genes can be efficiently delivered to cells of the peripheral nervous system using a replication incompetent HSV vector with mutations to ICP27, ICP4 and vmw65 following direct injection into a peripheral nerve.

Balb/c mice were inoculated by direct injection into sciatic nerve with 2-5ul of virus strain 1764/27-/4-/pR191acZ at titres of 1x 10⁷pfu/ml, 1x10⁸pfulml and 5x 10⁸pfu/ml. For inoculation, animals were anaesthetised, the sciatic nerve exposed, and virus suspension injected with a glass micropipette. The wound was then closed and animals allowed to recover. DRGs were removed 1 week after inoculation and fixed and stained with X-gal as above. In contrast to the results above, some gene delivery occurred at the lower two titres (e.g. <20 cells X-gal +ve at 1x10⁷ and 1x10⁸), and very high gene delivery efficiency could be achieved at a titre of 5x10⁸ with many hundreds to thousands of X-gal staining, lacZ+ve, cells being observed. Therefore, at sufficient titre, replication incompetent HSV can be used for highly efficient gene delivery to dorsal root ganglion cells following direct peripheral nerve inoculation of vector suspensions.

### Example 3. Genes can be efficiently delivered to cells of the peripheral nervous system using a replication incompetent HSV vector with mutations to ICP4 and vmw65 following direct injection into a peripheral nerve.

Balb/c mice were inoculated by direct injection into sciatic nerve with 2-5µl of virus strain 1764/4-/pR191acZ at a titre of 3x10⁸pfu/ml. For inoculation, animals were anaesthetised, the sciatic nerve exposed, and virus suspension injected with a glass micropipette. The wound was then closed and animals allowed to recover. DRGs were removed 1 week after inoculation and fixed and stained with X-gal as above. Similar to the results above in Example 2, many X-gal staining, lacZ +ve cells could be observed.

### Example 4. Replication incompetent HSV vectors expressing Cre recombinase direct efficient excision of intervening gene sequences in loxP containing transgenic mice.

Virus strain 1764/27-/4-/pR19Cre was constructed by replacement of the lac Z gene in virus strain 1764/27-/4-/pR191acZ with the Cre recombinase gene by homologous recombination with plasmid pR19Cre. Plasmid pR19Cre was constructed by replacement of the lacZ gene in plasmid pR19 (wagstaff et al 1998) with the Cre recombinase gene (Sterberg and Hamilton 1981). Modified Rosa26 mice (Mao et al 1999) which are a transgenic line containing lacZ inserted at a genomic position allowing constitutive expression but preceded by the neomycin resistance gene flanked by loxP sites which prevents lacZ expression in the absence of prior Cre recombinase mediated recombination were inoculated via the sciatic nerve with 2-5µl of either virus strain 1764/27-/4-/pR19Cre or virus strain 1764/27/4-/pR19GFP at titres of 3x10⁸pfu/ml. 2 days later DRGs were removed and fixed and stained with X-gal as above after observation for GFP fluorescence by fluorescence microscopy. DRGs from mice inoculated with virus strain 1764/27-/4-/pR19GFP showed no X gal staining as expected but did show abundant GFP fluorescence. Thus virus infection per se does not stimulate recombination at the loxP sites in Rosa26 mice and does not therefore allow lacZ expression. DRGs from mice inoculated with virus strain 1764/27-/4-/pR19Cre however, while showing no GFP fluorescence as expected showed abundant lacZ expression, in similar numbers of cells as GFP was expressed following inoculation with virus strain 1764/27-/4-/pR19GFP, indicating that Cre-mediated recombination at the loxP sites had occurred and therefore that functional Cre recombinase had been produced.

### References

Stevens 1987 Science 235; 1056-1059
Fink *et al*. 1996 Ann Rev Neurosci 19; 265-287
Goldstein and Weller 1988 J Virol 62; 196-205
Leib *et al*. 1989 J Virol 63; 759-768
Palella *et al*. 1988 Mol Cell Biol 8; 457-460
Ho and Mokarski 1988 Virology 167; 279-283
Lokensgard *et al*. 1994 J. Virol 68; 7148-7158.
Dobson *et al*. 1995 J. Virol. 69; 2264-2270
Goins *et al*. 1994 J. Virol 68; 2239-2252.
Ecob Prince *et al*. 1995 J. Gen Virol. 76; 1527-1532
Margolis *et al*. 1993 Virology 197; 585-592
Lachmann *et al*. 1996 J. Gen. Virol. 77;2575-2582
Lachmann and Efstathiou 1997 J. Virol 71; 3197-3207
Wilson *et al*. 1999 PNAS 96; 3211-3216
Thompson *et al*. 1998 Virus genes 1(3); 275-286
Meignier *et al*. 1988 J. Infect. Dis. 158; 602-614
Samaniego *et al*. 1998 J. Virol 72; 3307-3320
Krisky *et al*. 1998 Gene Therapy 5; 1593-1603
Thomas *et al*. 1999 J. Viro173; 7399-7409
MacFarlane *et al*. 1992 J. Gen. Virol. 73; 285-292
Howard *et al*. 1998 Gene Therapy 5; 1137-1147
Wagstaff *et al*. 1998 Gene Therapy 5; 1566-1570
Samaniego LA *et al.* J. Virol. (1995); 69: 5705-5715
Coffin RS and Latchman DS (1996). In: Genetic Manipulation of the Nervous System (DS Latchman Ed.) pp 99-114: Academic-- Press, London.
Coffin *RS, et al*. (1996) Gene Therapy, 3, 886-891.
Ace CI, *et al*. (1989) J. Virol., 63, 2260-2269.
Smith, IL *et al*., 1992, Virology 186: 74-86.
Rice, SA and Knipe DM., 1990, J. Virol 64: 1704-1715.
DeLuca NA *et al*. J. Virol. 1985; 56: 558-570.
MacFarlane M*, et al*. (1992) J. Gen. Virol., 73, 285-292.
Lokensgard JR, *et al*. (1994) J. Virol., 68, 7148-7158.
Gossen M and Bujard H, 1992, PNAS 89: 5547-5551.
Gossen M *et al*., 1995, Science 268: 1766-1769.
Smiley, J. R., and J. Duncan, 1997, J. Virol. 71: 6191-6193.

## Claims

1. Use of a replication incompetent herpes virus which:
(i) lacks at least one functional immediate early gene selected from genes encoding ICP4 and ICP27;
(ii) lacks a functional VMW65 gene due to a mutation in said gene which abolishes its transcriptional-activation activity; and
(iii) comprises a heterologous gene operably linked to the LAT P2 region, or a fragment thereof, and a non-LAT promoter, wherein said LAT P2 region or fragment thereof is capable of providing long-term expression capability to said non-LAT promoter;
in the manufacture of a medicament for use in treating or preventing a peripheral nervous system disorder by administering said medicament to a peripheral nerve by direct injection into said nerve.

2. A use according to claim 1 wherein said virus is a herpes simplex virus 1 or 2.

3. A use according to claim 1 or 2 wherein said virus lacks both a functional gene encoding ICP4 and a functional gene encoding ICP27.

4. A use according to any one of the preceding claims wherein said virus lacks functional genes encoding ICP0, ICP4, ICP22 and ICP27.

5. A use according to any one of the preceding claims wherein said LAT P2 region is an HSV2 LAT P2 region.

6. A use according to any one of claims 1 to 4 wherein said LAT P2 region is an HSV 1 LAT P2 region.

7. A use according to claim 6 wherein said HSV1 LAT P2 region is nucleotides 118866 - 120219 of HSV 1 strain 17+, or a fragment thereof.

8. A use according to any one of the preceding claims wherein said virus is a herpes virus amplicon vector.

9. A use according to any one of the preceding claims wherein said heterologous gene encodes a polypeptide or antisense RNA of therapeutic use.

10. A use according to claim 9 wherein said polypeptide or RNA is capable of modulating pain, stimulating nerve regeneration or preventing nerve degeneration.

11. A method of studying a peripheral nervous system disorder in a non-human aminal, which method comprises monitoring an effect of expression of a heterologous gene in a peripheral nerve, wherein said heterologous gene has been delivered to said nerve by direction injection of a replication incompetent herpes virus as defined in any one of claims 1 to 10 into said nerve, wherein said gene is implicated in a peripheral nervous system disorder and wherein said disorder is selected from motor neuron disease, pain, peripheral nerve damage and a neuropathy.

12. A method according to claim 11 wherein said gene encodes a protein selected from a neuropeptide and a growth factor.

13. A method according to claim 11 or 12 wherein said non-human animal models said disorder.

14. A method according to claim 13 wherein said animal is a transgenic animal.

15. A method according to any one of claims 11 to 14 wherein sensitivity to pain stimuli, nerve regeneration and nerve degeneration is monitored.

## Patentansprüche

1. Verwendung eines replikationsinkompetenten Herpesvirus, welchem/s:
(i) mindestens ein funktionelles Immediate-Early-Gen fehlt, ausgewählt aus Genen, die ICP4 und ICP27 codieren;
(ii) ein funktionelles VMW65-Gen fehlt aufgrund einer Mutation in diesem Gen, welche die Transkriptionsaktivierungs-Aktivität aufhebt; und
(iii) ein heterologes Gen, das operativ an die LAT P2-Region geknüpft ist, oder ein Fragment davon, und einen nicht-LAT-Promotor umfasst, wobei die LAT P2-Region oder ein Fragment davon zur Verleihung eines langfristigen Expressionsvermögens an den nicht-LAT-Promotor fähig ist;
in der Herstellung eines Medikaments zur Verwendung in der Behandlung oder Verhütung einer peripheren Nervensystemstörung druch Verabreichen des Medikaments an einen peripheren Nerv durch Direktinjektion in den Nerv.

2. Verwendung nach Anspruch 1, wobei das Virus ein Herpes-simplex-Virus 1 oder 2 ist.

3. Verwendung nach Anspruch 1 oder 2, wobei dem Virus sowohl ein funktionelles Gen, das ICP4 codiert, als auch ein funktionelles Gen, das ICP27 codiert, fehlt.

4. Verwendung nach einem der vorangehenden Ansprüche, wobei dem Virus funktionelle Gene fehlen, die ICP0, ICP4, ICP22 und ICP27 codieren.

5. Verwendung nach einem der vorangehenden Ansprüche, wobei die LAT P2-Region eine HSV2 LAT P2-Region ist.

6. Verwendung nach einem der Ansprüche 1 bis 4, wobei die LAT P2-Region eine HSV1 LAT P2-Region ist.

7. Verwendung nach Anspruch 6, wobei die HSV1 LAT P2-Region die Nukleotide 118866-120219 des HSV1-Stamms 17+, oder ein Fragment davon, umfasst.

8. Verwendung nach einem der vorangehenden Ansprüche, wobei das Virus ein Herpesvirus-Amplikonvektor ist.

9. Verwendung nach einem der vorangehenden Ansprüche, wobei das heterologe Gen ein Polypeptid oder eine Antisense-RNA von therapeutischem Nutzen codiert.

10. Verwendung nach Anspruch 9, wobei das Polypeptid oder die RNA zum Modulieren von Schmerz, Stimulieren der Nervenregeneration oder Verhüten einer Nervendegeneration fähig ist.

11. Verfahren zum Untersuchen einer peripheren Nervensystemstörung in einem nicht-menschlichen Tier, welches Verfahren das Überwachen einer Wirkung der Expression eines heterologen Gens in einem peripheren Nerv umfasst, wobei das heterologe Gen zu dem Nerv durch Direktinjektion eines replikationsinkompetenten Herpesvirus, wie in einem der Ansprüche 1 bis 10 definiert, in den Nerv transportiert worden ist, wobei das Gen mit einer peripheren Nervensystemstörung in Verbindung steht und wobei die Störung ausgewählt ist aus Motoneuron-Erkrankung, Schmerz, peripherer Nervenschädigung und einer Neuropathie.

12. Verfahren nach Anspruch 11, wobei das Gen ein Protein codiert, ausgewählt aus einem Neuropeptid und einem Wachstumsfaktor.

13. Verfahren nach Anspruch 11 oder 12, wobei das nicht-menschliche Tier ein Modell für die Störung bildet.

14. Verfahren nach Anspruch 13, wobei das Tier ein transgenes Tier ist.

15. Verfahren nach einem der Ansprüche 11 bis 14, wobei die Empfindlichkeit für Schmerzreize, die Nervenregeneration und die Nervendegeneration überwacht wird.

## Revendications

1. Utilisation d'un herpès virus réplication-incompétent, qui
(i) manque d'au moins un gène précoce immédiat fonctionnel choisi parmi les gènes codant pour ICP4 et ICP27 ;
(ii) manque d'un gène VMW65 fonctionnel en raison d'une mutation dans ledit gène qui abolit ladite activité d'activation de transcription ; et
(iii) comprend un gène hétérologue lié d'une manière opérationnelle à la région P2 de LAT, ou à un fragment de cette dernière, et un promoteur non-LAT, où ladite région P2 de LAT ou son fragment est capable de conférer une possibilité d'expression à long terme audit promoteur non-LAT ;
pour préparer un médicament destiné à une utilisation dans le traitement ou la prévention d'un trouble du système nerveux périphérique, par administration dudit médicament à un nerf périphérique par injection directe dans ledit nerf.

2. Utilisation selon la revendication 1, pour laquelle ledit virus est un virus herpès simplex 1 ou 2.

3. Utilisation selon la revendication 1 ou 2, pour laquelle ledit virus manque tant d'un gène fonctionnel codant pour ICP4 que d'un gène fonctionnel codant pour ICP27.

4. Utilisation selon l'une quelconque des revendications précédentes, pour laquelle ledit virus manque de gènes fonctionnels codant pour ICP0, ICP4, ICP22 et ICP27.

5. Utilisation selon l'une quelconque des revendications précédentes, pour laquelle la région P2 de LAT est une région P2 du LAT du HSV2.

6. Utilisation selon l'une quelconque des revendications 1 à 4, pour laquelle ladite région P2 de LAT est une région P2 du LAT du HSV1.

7. Utilisation selon la revendication 6, pour laquelle ladite région P2 du LAT du HSV1 est constituée des nucléotides 118866 - 120219 de la souche 17+ du HSV1, ou un fragment de ces nucléotides.

8. Utilisation selon l'une quelconque des revendications précédentes, pour laquelle ledit virus est un vecteur amplicon d'herpès virus.

9. Utilisation selon l'une quelconque des revendications précédentes, pour laquelle ledit gène hétérologue code pour un polypeptide ou un ARN antisens ayant une utilisation thérapeutique.

10. Utilisation selon la revendication 9, pour laquelle ledit polypeptide ou ARN est capable de moduler la douleur, de stimuler la régénération des nerfs ou de prévenir la dégénérescence des nerfs.

11. Procédé d'étude d'un trouble du système nerveux périphérique chez un animal non humain, lequel procédé comprend la surveillance d'un effet de l'expression d'un gène hétérologue dans un nerf périphérique, où ledit gène hétérologue a été délivré audit nerf par injection directe d'un herpès virus réplication-incompétent selon l'une quelconque des revendications 1 à 10 dans ledit nerf, ledit gène étant impliqué dans un trouble du système nerveux périphérique, et ledit trouble étant choisi parmi la sclérose latérale amyotrophique, la douleur, les lésions des nerfs périphériques et une neuropathie.

12. Procédé selon la revendication 11, dans lequel ledit gène code pour une protéine choisie parmi un neuropeptide et un facteur de croissance.

13. Procédé selon la revendication 11 ou 12, dans lequel ledit animal non humain modélise ledit trouble.

14. Procédé selon la revendication 13, dans lequel ledit animal est un animal transgénique.

15. Procédé selon l'une quelconque des revendications 11 à 14, dans lequel on surveille la sensibilité à des stimuli de douleur, à la régénération de nerfs et à la dégénérescence des nerfs.
